# EUROPEAN PATENT SPECIFICATION

(11) **EP 4 054 692 B1**
(45) Date of publication and mention of the grant of the patent: **27.05.2026**
(21) Application number: 20818674.2
(22) Date of filing: 30.06.2020
(51) Int. Cl.: A61M 25/01, A61M 25/00, A61M 25/09, A61M 25/092, A61M 25/16, A61M 25/06

(54) **CATHETER DEVICE**
KATHETERVORRICHTUNG
DISPOSITIF DE CATHÉTER

(43) Date of publication of application: 14.09.2022
(73) Proprietor: ProMedica Health System, Inc., Toledo, OH 43604 (US)
(72) Inventor: RAMANATHAN, P., Kasi, Toledo, OH 43604 (US); JUNG, Eugene, J., Toledo, OH 43604 (US)
(74) Representative: Gevers Patents
(86) International application number: PCT/US2020/040246
(87) International publication number: WO 2020/247964

(56) References cited:
- EP-A1- 0 505 686
- WO-A1-2018/023038
- US-A- 5 396 880
- US-A- 6 090 072
- US-A1- 2005 004 553
- US-A1- 2005 070 844
- US-A1- 2005 090 890
- US-A1- 2008 172 036
- US-A1- 2011 077 621
- US-A1- 2012 029 334
- US-A1- 2018 256 848
- US-A1- 2021 379 333

## Description

### FIELD

The present technology relates to catheter devices, including guide extension catheters that provide improved control of distal configurations thereof.

### INTRODUCTION

This section provides background information related to the present disclosure which is not necessarily prior art.

Guide catheters are used in nearly all of the approximately three million Percutaneous Coronary Intervention (PCI) procedures performed each year in the world. Percutaneous coronary intervention procedures are intended to clear blockages in coronary arteries that nourish the heart with blood. A blocked artery, if severe, can lead to irreversible heart muscle damage, stroke, or death if the blockage is left untreated. A guide catheter is designed to access coronary arteries during PCI so operators can deliver therapeutic devices, such as an angioplasty balloon or coronary stent, to treat the vessel blockage and restore blood flow to the heart. The performance of a guide catheter is critical to the success of a PCI procedure.

A guide catheter, a guidewire, and a stent mounted on a balloon catheter are the mainstays of PCI. While there are other niche devices used, the aforementioned three devices are used in almost every PCI procedure today. Even though tremendous improvements in coronary guidewire and stent design have occurred through the years, advances in guide catheter design have been minimal at best.

At the same time, guide catheters have decreased in diameter from 7 Fr or 8 Fr twenty or so years ago to 5 Fr or 6 Fr today. The smaller diameter helps to reduce complications at the vascular access site, near the groin or in the arm.

However, an unwanted consequence in reducing the guide catheter size is a reduction in support. The term support as used here describes the stability of guide catheter positioning at or near the coronary ostium, for example. It is essential that the guide catheter remains in position so that the guidewire and stent can be delivered to the treatment site. Loss of support describes a situation where the guide catheter backs out of position near the ostium as the operator tries to advance the guidewire or stent to the lesion in the coronary artery, for example. When this occurs, the operator must take his/her attention away from treating the blockage and attempt to restore the guide catheter into proper position near the ostium. Often, guide catheter backout recurs repeatedly during the same procedure, leading to operator frustration and lengthened procedure time. In addition, repeated catheter manipulation at or in the coronary artery can result in an injury to the vessel, which can create an adverse complication to the patient.

The concept of a guide extension catheter, in other words an inner catheter placed concentrically within an outer catheter, to enhance guide catheter support has been previously explored. Takahashi et al. ("New Method to Increase a Backup Support of Six French Guiding Coronary Catheter", Catheterization and Cardiovascular Interventions, 63:452-456, 2004) published data measuring the added support of an inner guide catheter extension within a conventional 6 Fr outer guide catheter. In the Takahashi study, the distal end of the inner catheter was extended past the distal end of the outer catheter into a model of a coronary vessel to evaluate catheter support. The results demonstrated that the above configuration offered improved support, enabling a 5 Fr inner and 6 Fr outer catheter system to exceed the amount of support of a larger guide catheter.

A number of devices in the art more broadly use a 2-in-1 catheter concept for applications beyond guide catheter extension devices. For example, Bowe (US7717899B2, US8401673B2) teaches of changing the shape of the catheter tip by rotating and translating the inner catheter relative to the outer catheter. In addition, Stys and Gainor (US20080172036A1, US20150119853A1) describe 2-in-1 catheters that can change shape and tip stiffness by manipulating the rotational and axial positions of one of the catheters relative to the other. However, having two separate, non-integrated catheters necessitate a cumbersome process in use.

Another 2-in-1 catheter concept by Root et al. (USRE45776E1) utilizes a rapid exchange style construction utilizing a pushrod attached to a short tubular member. Such devices extend into coronary arteries beyond the distal end of the guide catheter to enhance support. However, the use of guide catheter extension devices introduces an additional device into the procedure, adds significant cost, and adds to procedure time. In addition, separate extension devices take up space inside the lumen of the guide catheter reducing the space available for other devices. Importantly, as reported in the Manufacturer and User Facility Device Experience (MAUDE) database of the U.S. Food & Drug Administration, extension devices have been reported to cause vessel trauma.

The use of separate guide extension catheters, such as described above, can provide improved guide catheter support. However, such approaches, whether employing full length catheters or shorter rapid exchange devices with a pushrod element, are in essence, makeshift solutions. There accordingly remains a need to improve guide catheter design to overcome poor support while preserving improvements in catheter diameter reduction. Enhancing support, in turn, eliminates or reduces the frequent need to utilize a separate guide extension catheter.

Transcatheter Aortic Valve Replacement (TAVR) procedures could similarly benefit from such a 2-in-1 catheter device. For example, in post TAVR patients, the frame of TAVR valves makes engagement of the coronary artery challenging for PCI procedures. In these patients, physicians currently attempt to place the guide catheter close to the coronary artery ostium to be treated and then use an available guide extension catheter to engage the coronary artery to deliver balloons and stents. An integrated guide catheter/guide extension system would offer the same benefits along with improved workflow and a reduced number of devices needed to perform this complex and difficult procedure.

A further catheter device is known from EP 0 505 686 A1.

There consequently remains a need to more elegantly integrate guide extension catheters to provide the user with a simpler and easier set of controls to precisely and quickly change the catheter distal configuration. Such devices should more efficiently and cost effectively solve the problem of poor guide catheter support frequently experienced with currently available products and technologies in the art.

### SUMMARY OF THE INVENTION

The present invention is defined in claim 1 while preferred embodiments are set forth in the dependent claims. Associated surgical methods and methods of manufacture are also described herein to aid understanding the invention. These methods do not form part of the claimed invention.

### SUMMARY OF THE DISCLOSURE

The present technology includes articles of manufacture, systems, and processes that relate to a catheter device, including coronary catheters having integrated guide extension functionalities and improved support characteristics.

Ways of constructing and using catheter devices are provided, where such catheter devices include an outer tubular member, and inner tubular member, and a control mechanism. The outer tubular member has a proximal end and a shaped distal end. The inner tubular member has at least a portion thereof coaxially positioned within the outer tubular member. The inner tubular member includes a proximal end and a distal end. The control mechanism has a housing. The proximal end of the outer tubular member is immovably coupled to the housing. A luer or hub is coupled to the housing and seals an inner luminal space thereof. A fixed tubular segment has an end sealed to the luer or hub. The inner tubular member is slidably received over the fixed tubular segment and slidably received within the outer tubular member. The control mechanism further includes a slider immovably coupled to the inner tubular member to cause the slider and the inner tubular member to move in unison. The slider is provided as a stop ring configured to slide between a proximal stop and a distal stop of the control mechanism for defining a range of movement of the inner tubular member relative to the housing. The slider is configured to slide relative to the housing between a first position where the distal end of the inner tubular member is retracted into the distal end of the outer tubular member and a second position where the distal end of the inner tubular member is extended from the distal end of the outer tubular member.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Figure 1A shows an embodiment of a catheter device according to the present technology positioned within a patient's anatomy, where an inner tubular member is in a retracted state.
Figure 1B shows the embodiment of the catheter device according to Figure 1A, where the inner tubular member is in an extended state.
Figure 1C shows the embodiment of the catheter device according to Figure 1B, where the inner tubular member is being retracted.
Figure 2A shows an embodiment of a catheter device according to the present technology in a retracted and extended state.
Figure 2B shows an embodiment of a catheter device according to the present technology with a control mechanism including a slide handle in extended position.
Figure 3A shows an embodiment of a catheter device according to the present technology depicting inner components thereof, including a push wire in coiled form.
Figure 3B shows an embodiment of a catheter device according to the present technology depicting inner components thereof, including an actuator.
Figure 3C shows an embodiment of a catheter device according to the present technology depicting inner components thereof, including a plurality of actuators.
Figure 3D shows a cross-section of an embodiment of the catheter device according to Figure 3C depicting inner components thereof, including six actuators.
Figure 4A shows an embodiment of a control mechanism for an inner tubular member extension accordingly the present technology, including a rotating control mechanism with wire actuators and an inner tubular member in a retracted state.
Figure 4B shows the the embodiment of the control mechanism for the inner tubular member of Figure 4A with wire actuators in an extended state.
Figure 4C shows an alternative embodiment of a control mechanism for an inner tubular member extension according to the present technology, including a rotating control mechanism with coiled actuators in retracted state.
Figure 4D shows the inner tubular member of Figure 4C with coiled actuators in extended state.
Figure 5A shows a telescoping assembly according to the present technology having two concentric tubes, one able to slide over another (the outer tubular member is omitted for clarity), where the telescoping tubes are extended.
Figure 5B shows the telescoping assembly of Figure 5A, where the telescoping tubes are retracted.
Figure 6A is a cut-away view of an inner tubular member according to the present technology, where the inner tubular member in a retracted state.
Figure 6B shows the inner tubular member of Figure 6A in an extended state.
Figure 7A shows components of an inner tubular member assembly according to the present technology, where a partially compressible inner tubular member is in an extended state.
Figure 7B shows the partially compressible inner tubular member of Figure 7A in a retracted state.
Figure 7C shows an alternative embodiment of an inner tubular member assembly according to the present technology, including a telescoping mechanism showing concentric tubes, one sliding into another.
Figure 7D shows an alternative embodiment of an inner tubular member assembly according to the present technology, including a telescoping mechanism shown with concentric tubes separated and with a single push wire.
Figure 7E shows an alternative embodiment of an inner tubular member assembly according to the present technology, including a telescoping mechanism sliding shown with concentric tubes separated and with a coiled actuation mechanism.
Figure 8A shows an alternative embodiment of an actuation mechanism according to the present technology in a coiled configuration.
Figure 8B shows an alternative embodiment of an actuation mechanism according to the present technology with a multiple pulley arrangement.
Figure 8C shows a detailed view of the multiple pulley arrangement of Figure 8B.
Figure 9A shows an embodiment of a control mechanism according to the present technology having a rotating actuation mechanism.
Figure 9B shows an embodiment of a control mechanism according to the present technology having a sliding actuation mechanism.
Figure 10A shows an alternative embodiment of a pushrod mechanism according to the present technology integrated into a luer fitting.
Figure 10B shows the pushrod mechanism according to Figure 10A with the pushrod mechanism shown outside of the inner tubular member lumen.
Figure 10C shows the pushrod mechanism according to Figure 10A with the pushrod assembly in a retracted state (outer tubular member omitted for clarity).
Figure 10D shows the pushrod mechanism according to Figure 10A with the pushrod assembly in an extended state (outer tubular member omitted for clarity).
Figure 11A shows an embodiment of an inner tubular member according to the present technology, where a partially compressible inner tubular member is in an extended state.
Figure 11B shows an embodiment of an inner tubular member according to the present technology, where a partially compressible inner tubular member is in a retracted (shortened) state.
Figure 11C shows an embodiment of an inner tubular member according to the present technology, depicting a location of a control housing (outlined) when the inner tubular member is in an extended state.
Figure 11D shows an embodiment of an inner tubular member according to the present technology, depicting a location of a control housing (outlined) when the inner tubular member is in a retracted state.

### DETAILED DESCRIPTION

The present technology is drawn to catheter devices and uses thereof that include inner and outer tubular members and a control mechanism. The outer tubular member includes a proximal end and a shaped distal end and the inner tubular member includes a proximal end and a distal end. At least a portion of the inner tubular member is coaxially positioned within the outer tubular member. The control mechanism includes a housing having a means to seal an inner luminal space thereof. The control mechanism is coupled to the proximal end of the outer tubular member and the proximal end of the inner tubular member. The distal end of the inner tubular member can extend and retract from the shaped distal end of the outer tubular member by operation of the control mechanism.

The catheter device improves the state of the art in coronary guide catheters by providing for a low profile diameter, 6 Fr or less, guide catheter designed to offer enhanced support to prevent guide catheter backout or loss of support. In addition, the catheter device is configured to provide the above benefits in a single, convenient and easy to use catheter system based on a novel mechanism contained within or near the proximal end of the catheter. The catheter device offers enhanced workflow benefits, ease of use, and at the same time, eliminates the need for expensive ancillary devices. The extra devices needed in procedures today add clutter to the work space and consume valuable space inside the guide catheter. The space inside the present catheter device can instead be used for other purposes, for example, allowing room for a buddy wire technique.

Certain embodiments of the catheter device include an outer tubular member with a shaped distal end, a straight inner tubular member, and a control mechanism and its housing. The inner and outer tubular members are coaxially arranged with their respective proximal ends coupled to a control mechanism at or near the proximal end of the device. The control mechanism housing has an integrated luer fitting or means to seal the inner luminal space using an ancillary sealing means, such as a Touhy Borst fitting.

The control mechanism can be wholly or partially contained in the control mechanism housing near or at the proximal end of the catheter system. The control mechanism provides a means to extend the inner tubular member from inside the outer tubular member so it extends beyond the distal end of the outer tubular member. The control mechanism is also adapted to retract the inner tubular member so it is fully contained within the outer tubular member. The range of extension and retraction are controlled by stops in the control mechanism and define the maximum extension of the inner tubular member and the full range of travel of the inner tubular member relative to the distal end of the outer tubular member. The control mechanism and all of its parts are configured to be outside the inner tubular member so as to not utilize any space within the catheter system lumen, thus maximizing the available space within the inner lumen for delivering other devices such as guidewires, stent delivery catheters, etc.

The means of extending and controlling the inner tubular member movement include, but are not limited to manual, mechanical, electrical, electromagnetic and other means to extend the inner tubular member from within the outer tubular member and retract the inner tubular member into the outer tubular member. An example includes a manually operated control mechanism with an integrated luer that incorporates an external control ring and a means to convert rotational movement of the external control ring to a longitudinal movement of the inner tubular member using a mechanical system of a rotating knob, a gear set, pulley(s), and/or pushrod(s), etc. The aforementioned control mechanism is coupled to the inner tubular member thus translating movement of the external control ring to extend the inner tubular member from the outer tubular member and retract the inner tubular member into the outer tubular member.

The shaped distal end of the outer tubular member can be one of many existing shapes used in guide catheters. An example can be a Judkins right curve or an Amplatzer left curve or any of the standard Judkins or Amplatzer curves widely available. These and other curved shaped distal ends were developed to gain easier access to a specific coronary artery and to provide support for the catheter to remain in position during the procedure.

The outer tubular member is immovably affixed to the control mechanism and/or hub and the luminal space is sealed to prevent blood loss or air ingress/egress. A hub is an interface fitting designed to attach an accessory to the catheter in a sealed manner that prevents air leaks. The inner tubular member is coupled to the control mechanism, which, with operator manipulation, can extend the tip of the inner tubular member beyond the distal tip of the outer tubular member.

This catheter device provides the extra support and stability of a larger guide catheter or the combination of a standard guide catheter and guide extension catheter. The mechanical system of a rotating knob, a gear set, pulley(s), and/or pushrod(s), etc mentioned above describes the means of actuating the movement of the inner tubular member. Importantly, the actuation means, which can extend and retract the inner tubular member, acts without utilizing any space inside the inner tubular member inner lumen. Thus, the luminal space is preserved for other uses, such as inserting additional devices into the artery.

In summary, this catheter device offers the benefit of allowing a smaller vascular access site to reduce complications and a control mechanism that can extend the inner tubular member into the vasculature when needed, all in a single, easy to use catheter system. It does this in a novel way minimizing device length, and consequently, preserving the length a device inserted through the catheter device can be advanced into the vasculature. Another benefit is this design reduces clutter at the proximal end, where the operator must manipulate devices such as a guidewire or stent delivery catheter. The present technology can be used in a variety of applications such as interventional cardiology, interventional neurology and peripheral vascular intervention where traversing vasculature, providing guide catheter support, and offering a low device profile are needed.

Example embodiments of the present technology are provided with reference to the several figures enclosed herewith.

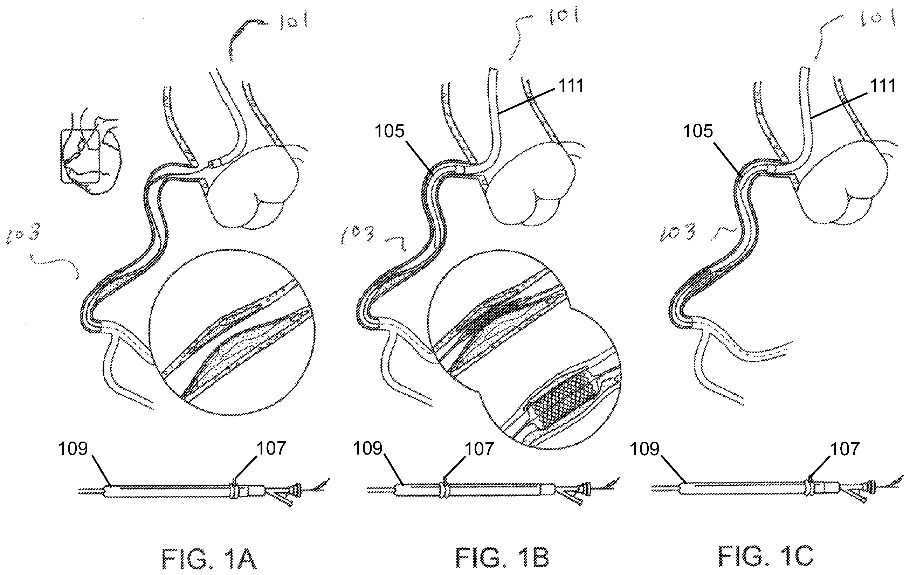

One embodiment of a catheter device 101 located in a coronary vessel 103 is shown in Figures 1A, 1B, and 1C. The catheter device 101 has an inner tubular member 105 that can be extended into the coronary vessel 103 to enhance guide catheter support, as shown in Figure 1B. In the lower part of each panel, the position of a slider 107, in a control mechanism 109, corresponds to the position of the inner tubular member 105 with respect to an outer tubular member 111. The slider 107 position and corresponding inner tubular member 105 position are shown in a fully retracted state (Figure 1A), a fully extended state (Figure 1B), and an intermediate state (Figure 1C). At the completion of the procedure, the extended inner tubular member 105 can be retracted and the guide catheter system withdrawn from the anatomy as shown in progress in Figure 1C.

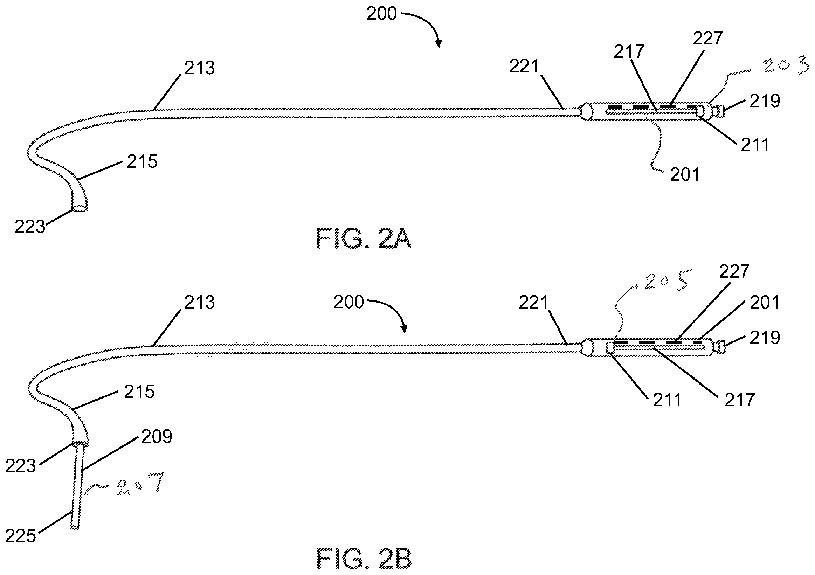

Figures 2A and 2B show another embodiment of a catheter device 200. In Figure 2A, a slider style control mechanism 201 is shown when an inner tubular member 209 is in a fully retracted position with a slider 211 in its most proximal position 203. An outer tubular member 213 is shown with a shaped distal end 215. In Figure 2B, the slider style control mechanism 201 is shown in its most distal position 205 corresponding to the inner tubular member 209 in its most extended position 207.

As shown in Figures 2A and 2B, the available range of movement of the inner tubular member 209 is limited by the design of the control mechanism 201, an open slot 217 in the control mechanism 201 precisely defines the longitudinal range of movement of the inner tubular member 209. This slot 217 feature defines the range of travel and frees the operator from having to take his/her eyes away from other tasks to manipulate the inner tubular member 209 position. In addition, in other embodiments a rail, slot, or other means can be incorporated to ensure only longitudinal range of movement occurs in a similar manner to that shown in Figures 2A and 2B. Use of a rail or slot 217 can preclude rotational movement of the control mechanism 201 and inner tubular member 209, which may confuse an operator manipulating the catheter device 200, thus possibly creating an unwanted distraction requiring the attention of the operator.

Markings shown at 227 can provide a unit of measure or scale for the user to see the amount of extension of the inner tubular member 209 extending from the outer tubular member 213. Such markings 227 can be integrated into the housing and/or applied onto the control mechanism 201. The markings 227 can include various indicia, including numbers, graduations, symbols, coloration, etc. In certain embodiments, the markings 227 directly correspond to a length that the inner tubular member 209 extends from the shaped distal end 215 of the outer tubular member 213 in positioning the slider 211 relative to the markings 227.

With further respect to the outer tubular member 213, as shown in Figures 2A and 2B, the outer tubular member 213 is configured as a catheter shaft having the shaped distal end 215 designed to facilitate access to a target vessel, such as a coronary artery ostium. Examples of the shaped distal end 215 can include a Judkins right or Amplatzer left guide configurations, among others. The outer tubular member 213 is sealed to prevent air ingress/egress. The outer tubular member 213 is also immovably affixed to the control mechanism 201 and/or a hub 219. The outer tubular member 213 can be a composite shaft tubing (not shown) comprised of a PTFE (trade name TEFLON) liner to serve as the innermost layer of the outer tubular member 213, a braid, and an overcoat to seal the braid into a cohesive structure. A stainless steel braid may be woven onto the aforementioned PTFE liner. The braid material may be made from any grade of stainless steel, such as 302, 304, or 316LV. Other metals such as nitinol are also contemplated for use. In addition, other materials can be used for the braid including polymeric materials like polyamide (trade name NYLON) or liquid crystal monofilament polymer (LCP). Non-ferrous and non-metallic braid material may be used to make the device MRI compatible. The "PIC" count of the braid may be varied to optimize the flexibility and torque characteristics of the outer tubular member 213. The PIC count can vary at the shaped distal end 215, where more flexibility may be desired, and at a proximal end 221 where greater stiffness may be desired. "PIC" refers to the amount of times the braiding crosses itself, in crosses per inch, for a woven pattern. A higher PIC count improves catheter shaft flexibility and a lower PIC count increases catheter shaft stiffness. The PIC count can be varied within a specified length to provide variable flexibility.

An overcoat can be applied to the braid and PTFE liner. The overcoat can be extruded onto the braided liner or it can be applied using separate segments of tubular material and fused together using a heat process with a shrink tubing cover. Other known methods of outer tubular member construction are anticipated. The overcoat can be comprised of any number of suitable biocompatible polymers familiar to those in the art. Polymers include polyether block amide or PEBA, also known by its trade name PEBAX, available from ARKEMA, which is available in several grades ranging in stiffness from a Shore D hardness of 27 and a flexural modulus of 12 MPA to a Shore D hardness of 69 or higher with a flexural modulus of 513 MPA or higher. Available grades of PEBAX range from soft to stiff, PEBAX 2533 to 7433. Other suitable materials such as thermoplastic urethanes, such as Pellathane, from Lubrizol can also be used. One example configuration includes a proximal shaft made from Pebax 7233, to provide axial stiffness, attached to a softer distal segment made of Pebax 3533, to reduce stiffness. Any combination of polymer segments can be used to optimize the catheter shaft flexibility and stiffness both at the distal segment, the proximal segment, and anywhere in between.

An extreme distal tip 223 of the outer tubular member 213 can be made atraumatic by forming it from a soft grade of polymer such as Pebax 2533 or 3533. The tip 223 can be fused together as described above. The extreme distal tip 223 of the outer tubular member 213 can be loaded with radiopaque material, such as barium sulfate, loaded into PEBAX at a mass or volume percentage of 20 percent or more to make the tip 223 highly visible under fluoroscopy. Other loading percentages, either by weight or volume, can make the 223 tip more or less visible under fluoroscopy.

The shaped distal end 215 of the outer tubular member 213 can be preformed into any number of desirable shapes. Shapes can include Judkins Right in 3, 4, 5 or the shape may be an AL-1 or AL-2 or AL-3. Other guide catheter tip shapes, defined by nomenclature understood by device operators, can be preformed into the shaped distal end 215. The shaped distal end 215 can be formed with a shaped forming mandrel inserted into the outer tubular member 213 and then baked at an elevated temperature for a specified period of time prior to assembly with the inner tubular member 209. Various temperature and oven bake times can be used to preform the shaped distal end 215 of the outer tubular member 213. This can be done prior to assembly with the inner tubular member 209 and is well understood by those familiar with the art.

In an alternative embodiment, the outer tubular member 213 can be deflectable. Thus, additional controls in the control mechanism 201 can be incorporated to actuate a pull wire/distal anchor ring assembly incorporated into the outer tubular member 213. The pull wire of the pull wire assembly may be sheathed in a PTFE liner to promote smooth actuation. The pull wire assembly and teflon sheath may be inserted through a dedicated lumen in the outer tubular member 213 wall. A single and double pull wire configuration is anticipated enabling both unidirectional and bidirectional steering. Any number of lumens could be incorporated into the outer tubular member 213 wall. For example, four lumens incorporated into the wall can enable four axis steering. It is also contemplated that the inner tubular member 209 can be likewise configured to enable deflection. Similarly, one or more lumens integrated into the wall of the inner tubular member 209 can house a pull wire mechanism to facilitate inner tubular member 209 deflection.

Either the inner tubular member 209 or the outer tubular member 213 can be deflectable. It is also contemplated that both the inner tubular member 209 and the outer tubular member 213 can be deflectable. This arrangement of deflectable inner and outer tubular members 209, 213 integrated with the control mechanism 201, for example the slotted configuration, ensures that deflection of the inner and outer members 209, 213, relative to the other, remains unchanged. For example, the outer tubular member 213 can be designed to deflect in a posterior and anterior direction, while the inner tubular member 209 can be configured to deflect exactly 90 degrees from the outer tubular member 213, resulting in movement in a superior and inferior direction. Thus, precise and repeatable placement of the catheter device extreme distal tip 223 within the anatomy is possible.

With further respect to the inner tubular member 209, the following aspects can be considered. The inner tubular member 209 can include an assembly designed to move the inner tubular member 209 relative to the outer tubular member 213. In this way, a distal end 225 of the inner tubular member 209 can extend past the shaped distal end 215 of the outer tubular member 213. When used in the body, the distal tip 223 of the outer tubular member is positioned near or at the ostium of a coronary artery, then the distal end 225 of the inner tubular member 209 is advanced into the coronary artery to enhance catheter support. It is also contemplated that the catheter device 200 can be used in other areas of the body. The inner tubular member 209 is designed to extend from 10 to 40 cm past the distal end of the outer tubular member, depending on the control mechanism 201 position. However, the extendable range can be made any other length to better accommodate specific applications. The control mechanism 201 can be immovably coupled to the inner tubular member 209 to enable an operator to precisely control a length the inner tubular member 209 extends from the outer tubular member 213.

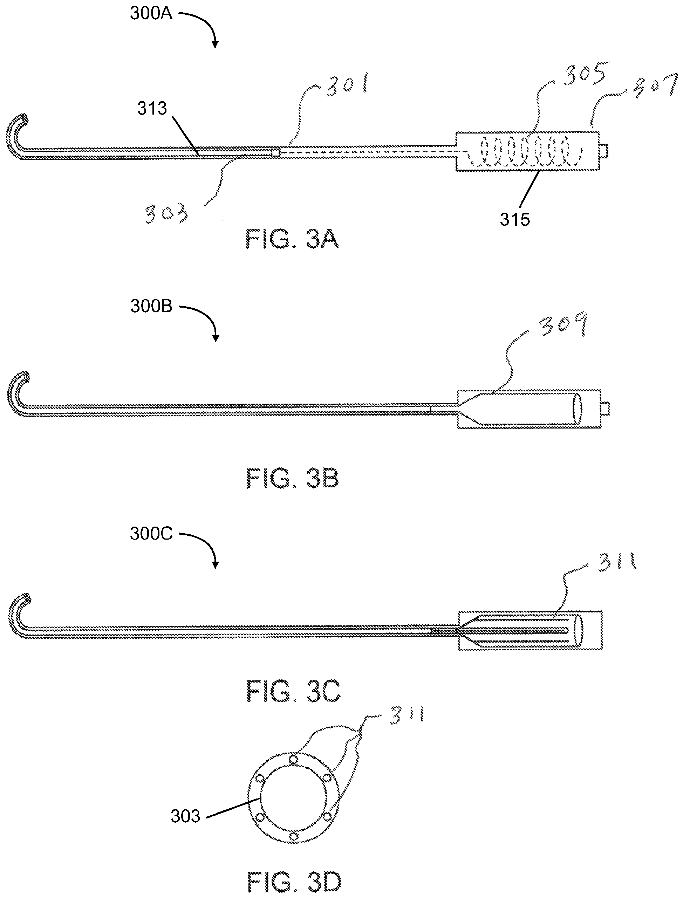

Other embodiments of catheter devices 300A, 300B, and 300C are shown in Figures 3A, 3B, and 3C, which depict views through an outer tubular member 301 drawn to reveal different embodiments of an inner tubular member 303 and other internal components. The inner tubular member 303 can include a tubular portion 313, a wire 305, which can also be a cable, coupled to both the tubular portion 313 and a control mechanism 307. The control mechanism 307 in Figure 3A shows a larger control mechanism housing 315. Figure 3A shows the wire 305 as a coiled wire providing a compact means to actuate the inner tubular member 303 for extension and retraction relative to the outer tubular member 301. Figure 3B shows an alternative embodiment with a wire 309, which can also be a cable. Figure 3C shows six wires 311, which can also be cables, arranged radially around the inner tubular member. Figure 3D represents a cross-sectional view showing the radial arrangement of wires 311, which can also be cables, around the inner tubular member 303. Any number of wires 311 or cables, etc. can be utilized as part of the control mechanism 307 to actuate movement of the inner tubular member 303. The actuation means, described later, is coupled to the wire(s) 311, which can also be cable(s), etc. to facilitate inner tubular member 303 extension or retraction from within the outer tubular member 301.

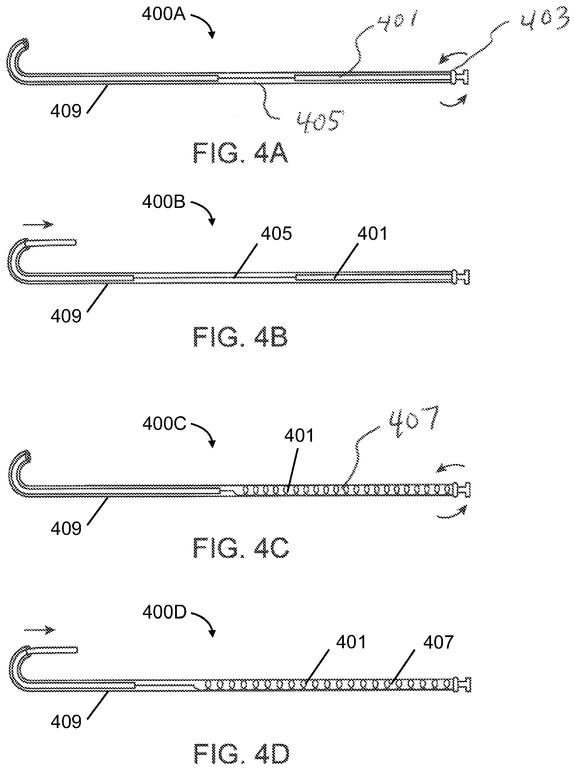

Figures 4A, 4B, 4C, and 4D show alternate embodiments of catheter devices 400A, 400B, 400C, 400D, each having a low profile or small diameter control mechanism 403 compared with the embodiments of Figures 3A-D. A see through view of an outer tubular member 409 shows the inner tubular member assembly 401 more clearly. The inner tubular member assembly 401 is coupled to the control mechanism 403 via a wire or cable in straight 405 or coiled form 407. Means of actuation are illustrated in other figures.

Figure 4A shows a rotating control mechanism 403, the movement highlighted by curved arrows, with one or more wires 405, which can also be cables, and the inner tubular member 401 in the retracted state. Figure 4B shows the inner tubular member 401 in Figure 4A with the wires 405 in an extended state. Figure 4C shows an alternative embodiment with a rotating control mechanism 403 with one or more coiled wires 407 in a retracted state. Figure 4D shows the inner tubular member in Figure 4C with the coiled wires 407 in an extended state. The coiled wires 407 can be used to reduce the needed space inside the control mechanism 403, making it possible to shorten the housing of the control mechanism 403.

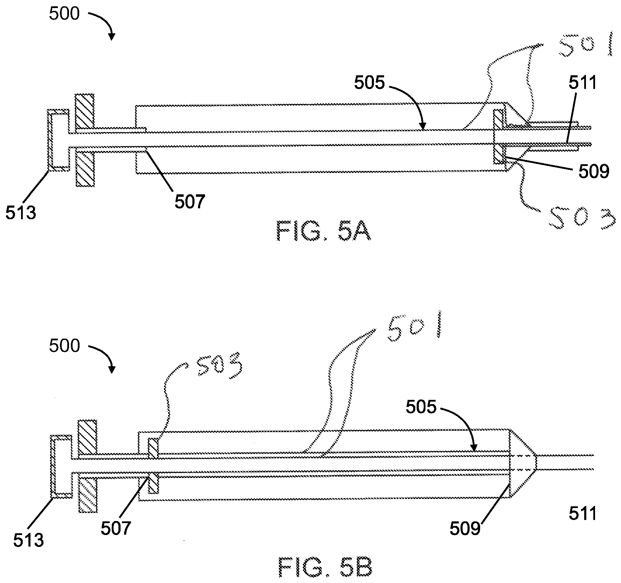

Figures 5A and 5B show an embodiment of a catheter device 500 incorporating an inner tubular member 511 having a telescoping feature 505 and a proximal stop 507 and a distal stop 509 defining a range of movement for the inner tubular member 511. The telescoping feature 505 is comprised of a plurality of concentric tubular segments 501 designed to slide over each other. This telescoping feature 505 reduces device length. The telescoping feature 505 of the inner tubular member 511 includes at least one fixed tubular segment 501 sealed to a luer or hub 513 and at least one slidable tubular segment 501. It is anticipated that one or more slidable telescoping segments 501 can be incorporated in the inner tubular member 511 assembly. A stop ring 503 is immovably coupled to one of the tubular segments 501 of the inner tubular member 511 and defines the range of movement of the inner tubular member 511. The stop ring 503 can be affixed to the inner tubular member 511 by swaging, crimping, heat bonding, adhesive bonding, and/or any other means of fastening components together. Figure 5A shows a slidable inner tubular member 511 in a distal most position correlating to the maximum extension of the inner tubular member 511 beyond the outer tubular member (not shown). Figure 5B illustrates the position of the slidable inner tubular member 511 when fully retracted.

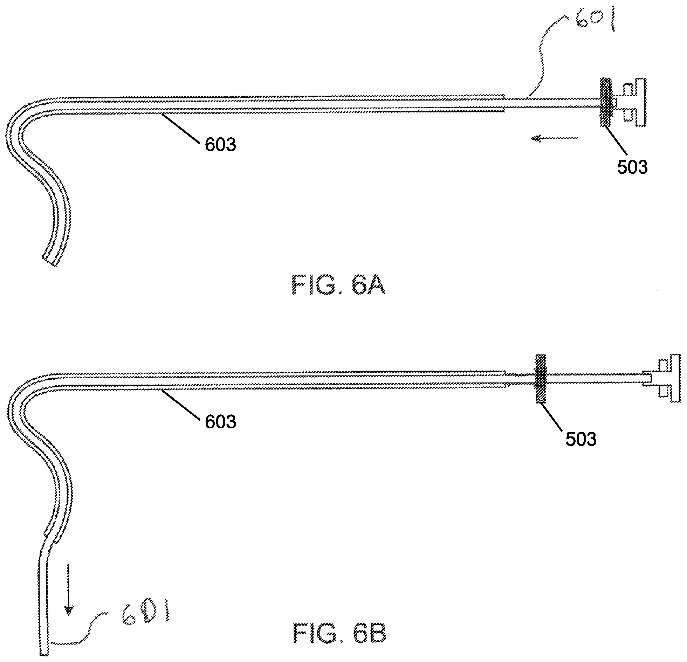

The telescoping feature 505 of the inner tubular member 511 in the embodiment of Figure 5 is shown inside an outer tubular member 603 in Figure 6. The control mechanism housing is omitted for clarity. The stop ring 503 shows the position of the inner tubular member 601 relative to the outer tubular member 603 in their respective positions. In Figure 6A the slidable inner tubular segment of the inner tubular member 601 is shown in a retracted state and in Figure 6B the slidable inner tubular segment of the inner tubular member 601 is shown in an extended state.

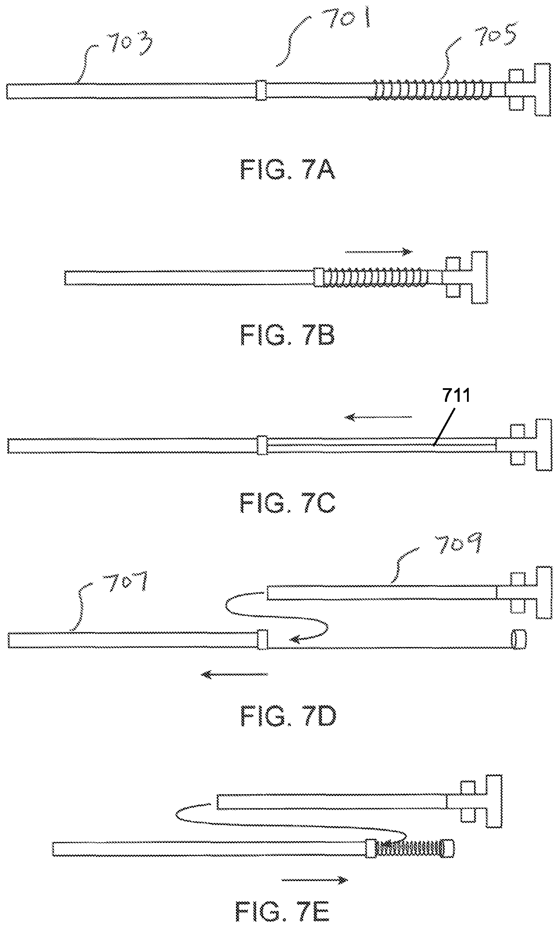

As shown in Figures 7A, 7B, 7C, 7D, and 7E, embodiments of an inner tubular member 701 can include a polymer tube 703 and a coil over tube subassembly 705. As shown in Figure 7A, the inner tubular member 701 is in an extended state. Figure 7B shows the compressible coil over tube subassembly 705 in a compressed state when the inner tubular member is in a retracted state. Figure 7C shows an alternative embodiment replacing the coil over tube assembly 705 in Figure 7A with a wire 711. In addition, as shown in Figures 7D and 7E, a telescoping structure, made from at least two separate inner tubular segments (e.g., as shown for Figures 6A-B), including at least one slidable inner tubular segment 707 and at least one non-slidable inner tubular segment 709, that can be advanced or retracted one over the other. The mechanism of actuation can compress the length of the inner tubular member 701 when retracted into the control mechanism. It is anticipated that a plurality of slidable tubes using a telescoping arrangement may be utilized to reduce the control housing length when the inner tubular member 701 is fully retracted.

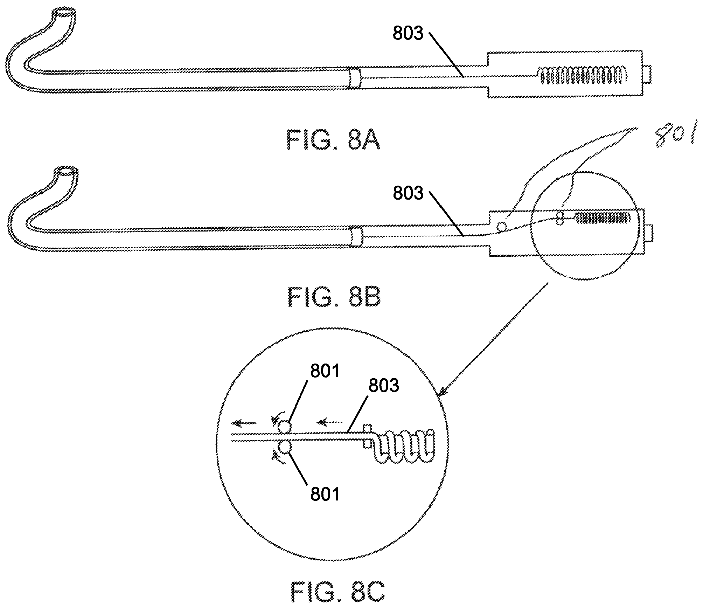

Figures 8A, 8B, and 8C show an embodiment using a plurality of pulleys 801 operating with at least one wire 803 to facilitate the extension or retraction of the inner tubular member and can be incorporated into the control mechanism (not shown) to facilitate extension or retraction of the inner tubular member relative to the outer tubular member.

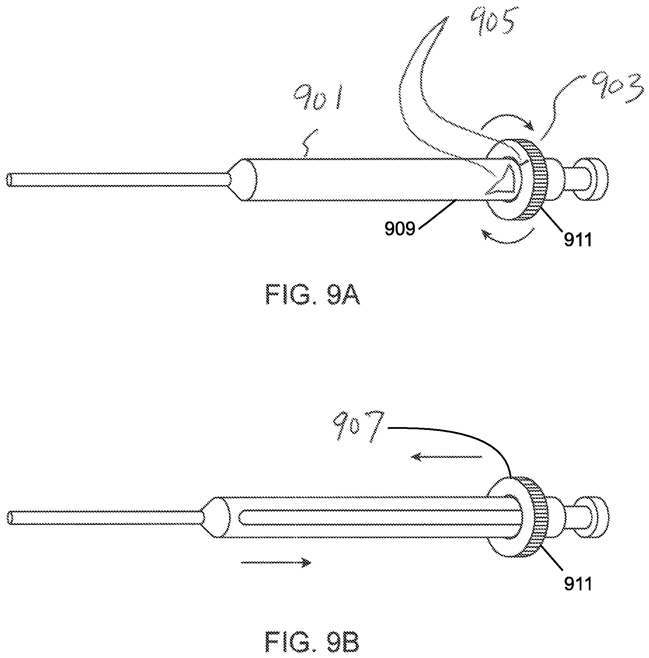

Figure 9A shows an embodiment of a control mechanism 901 with a rotating control ring 903. The rotating ring 903 has a series of undulations 911 on a surface thereof to improve grip. Markings 905 on the rotating ring 903 and outer control mechanism housing 909 can provide a visual indication of an extent of inner tubular member extension. Alternatively, rather than symbology, the markings can include numerical units to serve the same function. Figure 9B shows a control mechanism 901 using an aforementioned slide mechanism 907 (e.g., Figures 1A-C, 2A-B) that also incorporates undulations 911 to promote improved grip.

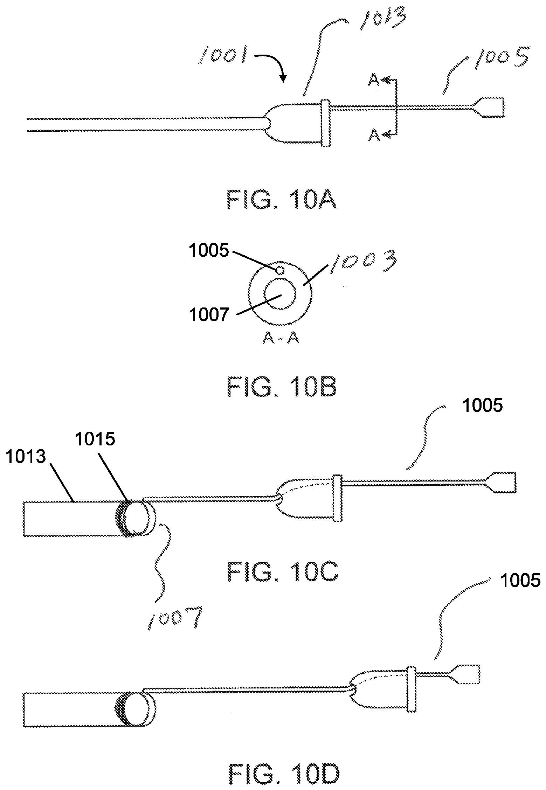

Figures 10A, 10B, 10C, and 10D show an alternative embodiment of a control mechanism 1001 including a pushrod 1005 routed outside the inner tubular member lumen 1007, which is depicted in Figure 10B showing an end view of the control mechanism 1001 shown from the perspective of the cut line A-A in Figure 10A. In other words, the pushrod 1005 enters the luer or hub 1003 outside the lumen 1007. This enables the luminal space 1007 to be utilized by other devices. The pushrod 1005 is coupled to the inner tubular member 1013 via an anchor ring 1015 or other means to immovably fasten the pushrod 1005 to the inner tubular member 1013. The pushrod 1005 actuates the inner tubular member 1013 movement. Figure 10C shows the position of the pushrod 1005 maximally extended toward the operator and the inner tubular member 1013 is fully retracted. When the pushrod 1005 is maximally advanced into the luer 1003 the inner tubular member 1013 is fully extended past the outer tubular member (not shown).

Another embodiment can be comprised of a plurality of inner tubular segments concentrically arranged to slide one over another, so the inner tubular member becomes shorter or longer when actuated by the pushrod 1005 or other actuating means; e.g., Figures 5A-B, 6A-B. The pushrod 1005 can be located outside the inner lumen 1007 to preserve the luminal space for delivering guidewires and catheters. The pushrod 1005 can be coupled to the inner tubular member by different means, such as an anchor ring 1015 immovably affixed to the inner tubular member. Other configurations coupling the pushrod 1005 to the inner tubular member are possible.

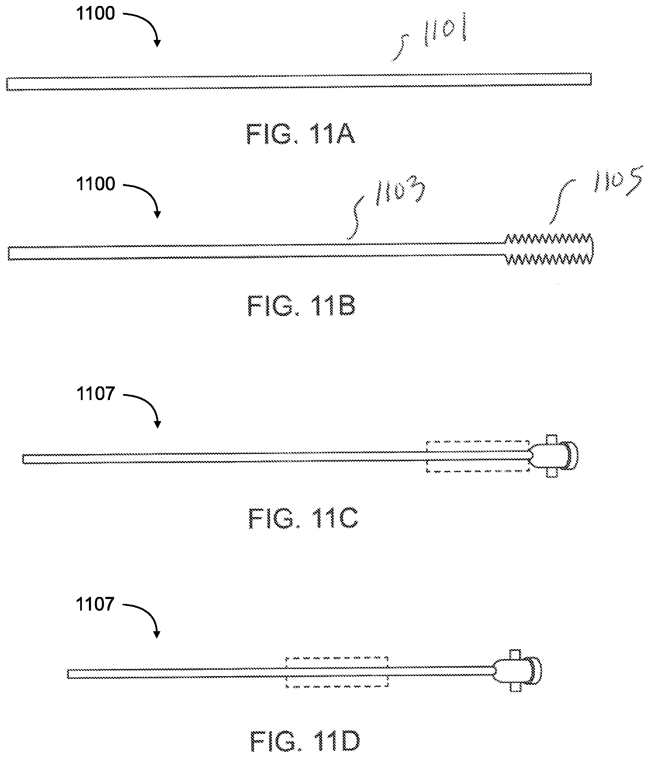

In yet another embodiment shown in Figures 11A and 11B, the inner tubular member 1100 can have both an extended and retracted state. This is accomplished by the use of a compressible bellows tube, where the inner tubular member 1100 may be comprised of a partially compressible member formed from a bellows structure. Figure 11A shows the partially compressible member in a fully extended state 1101. Figure 11B shows the partially compressible member in a compressed state 1103. A compressible bellows segment 1105 of the inner tubular member 1100 can reside in the control mechanism, joined or fused to a incompressible member that can extend past the control mechanism. In this way, a shorter control mechanism is possible. The advantage of a compressible, or collapsible, inner tubular member 1100 is to reduce the total catheter device length. A collapsible section can range from a few millimeters to over 40 centimeters. More preferably, it is intended to collapse from 5-20 centimeters.

In an alternative embodiment shown in Figures 11C and 11D, an incompressible inner tubular member 1107 can slide, proximal to distal, within the outer tubular member (not shown) so a portion of the inner tubular member extends from the outer tubular member. This simple arrangement offers the benefit of a lower manufacturing cost.

Catheter devices described herein can be used in various ways. Among many available guide catheter shapes, the device operator can select one which he/she believes will result in adequate coaxial support for a successful intervention. The guide catheter can be used to engage the coronary artery in a standard fashion, as one would with any other type of guiding catheter that is currently available for use. For example, to engage the right coronary artery, one would take a standard 6Fr JR4 shape and advance the guide catheter to the ascending aorta with an 0.035 inch guidewire. Then, one would gently touch the aortic valve with the guide catheter then pull the guide catheter back with the right hand on the proximal portion, and subsequently, with the left (and right) hands apply a clockwise torque to turn the catheter into the right coronary artery to engage the ostium. Similarly, for the left coronary artery, one would take a standard shape guide catheter, such as a 6 Fr JL4 or EBU 4, and insert it over a 0.035 inch guidewire. The guide catheter and guidewire may be advanced in the ascending aorta, at which time the guidewire is removed. The guide catheter is advanced into the left coronary artery to engage the ostium.

For subsequent intervention, after the patient is adequately anticoagulated, an 0.014 inch wire is carefully advanced into the coronary artery to cross the stenotic lesion. Once the wire is past the lesion, one can advance balloon and stent catheters to treat the lesion. However, there may not be adequate support for the delivery of the balloon/stent catheter system. In this case, there are standard practices that can be employed such as: Use of a buddy wire system, further modification of the lesion with a balloon, use of a guide catheter extension, changing to a more supportive guidewire, changing to a larger guide catheter or a differently shaped guide catheter that may provide more support. These optional solutions are less than ideal, all requiring additional equipment, while disrupting the smooth workflow of an intervention. A better solution is needed, one that does not require the use of any additional equipment, which is important from a workflow, patient safety, and cost standpoint. An ideal solution would already be built into the guide catheter.

The method outlined below describes how the present technology can utilize a catheter device having a novel guide catheter system to provide improved treatment methods. The inner tubular member, in its initial configuration enters the body so its distal end does not protrude from the outer tubular member. The catheter device, in this initial configuration, is used to engage the coronary artery as described above. The lesion in question can be crossed with an 0.014 inch guidewire in a standard fashion as described above. When there is difficulty advancing the balloon/stent delivery system, or if it is anticipated that it may be difficult to advance, then the inner tubular member of the present technology can be extended into the vasculature to enhance guide catheter support.

The catheter device can be inserted into a vascular access site, into the femoral artery, near the groin using a technique well known for those in the art. Alternatively, the device may be inserted into the radial artery at the wrist of the patient. In both femoral or radial techniques, the catheter device is advanced through the vasculature until it reaches the ostium of a coronary artery. Markers on the catheter shaft can indicate the position of the catheter tip in the body and can consequently reduce the use of radiation in positioning the catheter.

Using the catheter device, an operator would first advance the balloon catheter about 10 to 15 mm into the coronary artery with his/her right hand, keeping the 0.014 inch guidewire in place with his/her left hand. The advanced balloon catheter may then serve as a rail to safely extend the inner tubular member of the catheter device. With the balloon catheter advanced in place down the treated vessel, then using the left hand, the inner guide catheter can be extended from the catheter device by manipulation of a control mechanism. The control mechanism acting on the inner tubular member, enabling both advancement into the artery or retraction into the outer tubular member, the means of actuation not utilizing space within the lumen of the inner tubular member thus enabling the use of the lumenal space for other purposes. This technique offers an extra measure of safety in preventing vessel dissection.

One method to extend the inner tubular member is for one to use his/her left hand to turn a small knob clockwise (for embodiment with rotating ring control mechanism), while holding the guidewire and balloon catheter with the right hand. Another method would be to extend the inner tubular member using a sliding mechanism with the left hand while holding the balloon and guidewire with the right hand.

Once the inner tubular member has been advanced into the coronary artery sufficient to provide support, then the intervention can be completed in typical fashion. To retract the extended inner tubular member when the intervention is complete, one would turn the knob counterclockwise or retract the sliding mechanism back using the left hand, depending on the embodiment. The other equipment used to perform the procedure would then be retracted and the access site closed to complete the procedure.

## Claims

1. A catheter device comprising:
an outer tubular member (603) having a proximal end (221) and a shaped distal end (215);
an inner tubular member (601) having at least a portion thereof coaxially positioned within the outer tubular member (603), the inner tubular member (601) including a proximal end and a distal end (225); and
a control mechanism (201) having a housing, the proximal end (221) of the outer tubular member (603) being immovably coupled to the housing,
wherein:
a luer or hub (513) is coupled to the housing and seals an inner luminal space thereof;
a fixed tubular segment (501) has an end sealed to the luer or hub (513), wherein the inner tubular member (601) is slidably received over the fixed tubular segment (501) and slidably received within the outer tubular member (603);
the control mechanism (201) further includes a slider (211) immovably coupled to the inner tubular member (601) to cause the slider (211) and the inner tubular member (601) to move in unison, the slider (211) being provided as a stop ring (503) configured to slide between a proximal stop (507) and a distal stop (509) of the control mechanism (201) for defining a range of movement of the inner tubular member (601) relative to the housing; and
the slider (211) is configured to slide relative to the housing between a first position where the distal end (225) of the inner tubular member (601) is retracted into the distal end (215) of the outer tubular member (603) and a second position where the distal end (225) of the inner tubular member (601) is extended from the distal end (215) of the outer tubular member (603).

2. The catheter device of Claim 1, wherein an entirety of the control mechanism (201) is positioned outside of a lumen of the inner tubular member (601).

3. The catheter device of Claim 1, wherein the shaped distal end (215) of the outer tubular member (603) includes at least one curve.

4. The catheter device of Claim 1, wherein the shaped distal end (215) of the outer tubular member (603) includes one of a Judkins right curve and an Amplatzer left curve.

5. The catheter device of Claim 1, wherein the luer or hub (513) is configured as an interface fitting designed to attach an accessory to the catheter device in a sealed manner that prevents air leaks.

6. The catheter device of Claim 1, wherein the outer tubular member (603) changes in flexibility between the proximal end (221) and the distal end (215) thereof.

7. The catheter device of Claim 1, wherein the outer tubular member (603) and the fixed tubular segment (501) remain stationary relative to the housing when the slider (211) and the inner tubular member (601) are slid relative to each of the housing, the fixed tubular segment (501), and the outer tubular member (603).

8. The catheter device of Claim 1, wherein one of a guide wire, a balloon catheter, or a stent catheter is received axially through each of the luer or hub (513), the fixed tubular segment (501), and the inner tubular member (601).

## Patentansprüche

1. Eine Kathetervorrichtung, umfassend:
ein äußeres rohrförmiges Element (603), das ein proximalen Ende (221) und ein geformtes distales Ende (215) aufweist;
ein inneres rohrförmiges Element (601), das mindestens einen Abschnitt davon koaxial innerhalb des äußeren rohrförmigen Elements (603) positioniert aufweist, wobei das innere rohrförmige Element (601) ein proximales Ende und ein distales Ende (225) einschließt; und
einen Steuermechanismus (201), der ein Gehäuse aufweist, wobei das proximale Ende (221) des äußeren rohrförmigen Elements (603) unbeweglich mit dem Gehäuse gekoppelt ist,
wobei:
ein Luer oder Hub (513) mit dem Gehäuse gekoppelt ist und einen inneren Lumenraum davon abdichtet;
ein fixes rohrförmiges Segment (501) ein Ende aufweist, das mit dem Luer oder Hub (513) abgedichtet ist, wobei das innere rohrförmige Element (601) über dem fixen rohrförmigen Segment (501) verschiebbar aufgenommen ist und innerhalb des äußeren rohrförmigen Elements (603) verschiebbar aufgenommen ist;
der Steuermechanismus (201) weiter einen Schieber (211) einschließt, der unbeweglich mit dem inneren rohrförmigen Element (601) gekoppelt ist, um zu bewirken, dass sich der Schieber (211) und das innere rohrförmige Element (601) gemeinsam bewegen, wobei der Schieber (211) als Anschlagring (503) bereitgestellt ist, der so konfiguriert ist, dass er zwischen einem proximalen Anschlag (507) und einem distalen Anschlag (509) des Steuermechanismus (201) gleitet, um einen Bewegungsbereich des inneren rohrförmigen Elements (601) in Bezug auf das Gehäuse zu definieren; und
der Schieber (211) so konfiguriert ist, dass er in Bezug auf das Gehäuse zwischen einer ersten Position, in der das distale Ende (225) des inneren rohrförmigen Elements (601) in das distale Ende (215) des äußeren rohrförmigen Elements (603) zurückgezogen ist, und einer zweiten Position, in der das distale Ende (225) des inneren rohrförmigen Elements (601) aus dem distalen Ende (215) des äußeren rohrförmigen Elements (603) herausgefahren ist, gleitet.

2. Die Kathetervorrichtung nach Anspruch 1, wobei der gesamte Steuermechanismus (201) außerhalb eines Lumens des inneren rohrförmigen Elements (601) positioniert ist.

3. Die Kathetervorrichtung nach Anspruch 1, wobei das geformte distale Ende (215) des äußeren rohrförmigen Elements (603) mindestens eine Krümmung einschließt.

4. Die Kathetervorrichtung nach Anspruch 1, wobei das geformte distale Ende (215) des äußeren rohrförmigen Elements (603) eines einschließt von einer Judkins-Rechtskrümmung und einer Amplatzer-Linkskrümmung.

5. Die Kathetervorrichtung nach Anspruch 1, wobei der Luer oder Hub (513) als Schnittstellenformstück konfiguriert ist, das dazu konstruiert ist, ein Zubehörteil auf eine abgedichtete Weise, die Lufteckage verhindert, an der Kathetervorrichtung zu befestigen.

6. Die Kathetervorrichtung nach Anspruch 1, wobei sich das äußere rohrförmige Element (603) in Flexibilität zwischen dem proximalen Ende (221) und dem distalen Ende (215) davon ändert.

7. Die Kathetervorrichtung nach Anspruch 1, wobei das äußere rohrförmige Element (603) und das fixe rohrförmige Segment (501) in Bezug auf das Gehäuse stationär bleiben, wenn der Schieber (211) und das innere rohrförmige Element (601) in Bezug auf das Gehäuse, das fixe rohrförmige Segment (501) und das äußere rohrförmige Element (603) verschoben werden.

8. Die Kathetervorrichtung nach Anspruch 1, wobei eines von einem Führungsdraht, einem Ballonkatheter oder einem Stentkatheter durch jedes des Luer oder Hubs (513), des fixen Rohrsegments (501) und des inneren rohrförmigen Elements (601) axial aufgenommen wird.

## Revendications

1. Un dispositif de cathéter comprenant :
un élément tubulaire externe (603) présentant une extrémité proximale (221) et une extrémité distale profilée (215) ;
un élément tubulaire interne (601) présentant au moins une partie de celui-ci positionnée coaxialement à l'intérieur de l'élément tubulaire externe (603), l'élément tubulaire interne (601) incluant une extrémité proximale et une extrémité distale (225) ; et
un mécanisme de commande (201) présentant un boîtier, l'extrémité proximale (221) de l'élément tubulaire externe (603) étant couplée de manière immobile au boîtier,
dans lequel :
un luer ou moyeu (513) est couplé au boîtier et scelle un espace luminal interne de celui-ci ;
un segment tubulaire fixe (501) présente une extrémité scellée au luer ou au moyeu (513), dans lequel l'élément tubulaire interne (601) est reçu de manière coulissante sur le segment tubulaire fixe (501) et reçu de manière coulissante à l'intérieur de l'élément tubulaire externe (603) ;
Le mécanisme de commande (201) inclut en outre un curseur (211) couplé de manière immobile à l'élément tubulaire interne (601) pour amener le curseur (211) et l'élément tubulaire interne (601) à se déplacer à l'unisson, le curseur (211) étant prévu comme une bague d'arrêt (503) configurée pour coulisser entre une butée proximale (507) et une butée distale (509) du mécanisme de commande (201) pour définir une plage de mouvement de l'élément tubulaire interne (601) par rapport au boîtier ; et
le curseur (211) est configuré pour glisser par rapport au boîtier entre une première position où l'extrémité distale (225) de l'élément tubulaire interne (601) est rétractée dans l'extrémité distale (215) de l'élément tubulaire externe (603) et une seconde position où l'extrémité distale (225) de l'élément tubulaire interne (601) est étendue à partir de l'extrémité distale (215) de l'élément tubulaire externe (603).

2. Le dispositif de cathéter selon la revendication 1, dans lequel une intégralité du mécanisme de commande (201) est positionnée à l'extérieur d'une lumière de l'élément tubulaire interne (601).

3. Le dispositif de cathéter selon la revendication 1, dans lequel l'extrémité distale profilée (215) de l'élément tubulaire externe (603) inclut au moins une courbe.

4. Le dispositif de cathéter selon la revendication 1, dans lequel l'extrémité distale profilée (215) de l'élément tubulaire externe (603) inclut une courbe droite de Judkins et une courbe gauche d'Amplatzer.

5. Le dispositif de cathéter selon la revendication 1, dans lequel le luer ou le moyeu (513) est configuré en tant que raccord d'interface conçu pour fixer un accessoire au dispositif de cathéter de manière étanche qui empêche des fuites d'air.

6. Le dispositif de cathéter selon la revendication 1, dans lequel l'élément tubulaire externe (603) change de flexibilité entre l'extrémité proximale (221) et l'extrémité distale (215) de celui-ci.

7. Le dispositif de cathéter selon la revendication 1, dans lequel l'élément tubulaire externe (603) et le segment tubulaire fixe (501) restent stationnaires par rapport au boîtier lorsque le curseur (211) et l'élément tubulaire interne (601) sont glissés par rapport à respectivement chacun du boîtier, du segment tubulaire fixe (501) et de l'élément tubulaire externe (603).

8. Le dispositif de cathéter selon la revendication 1, dans lequel l'un d'un fil guide, d'un cathéter à ballonnet ou d'un cathéter à stent est reçu axialement à travers chacun du luer ou du moyeu (513), du segment tubulaire fixe (501) et de l'élément tubulaire interne (601).
